# EUROPEAN PATENT APPLICATION

(11) **EP 4 674 452 A1**
(43) Date of publication of application: **07.01.2026**
(21) Application number: 25185322.2
(22) Date of filing: 25.06.2025
(51) Int. Cl.: A61M 5/20, A61M 5/32, A61M 5/24, A61M 5/31, A61M 5/315

(54) **AUTO-INJECTOR**

(30) Priority: 05.07.2024 US 202418764773
(71) Applicant: Lent Innovations LLC, Milwaukee, Wisconsin 53202 (US)
(72) Inventor: Lent, Anne M., Milwaukee, WI 53202 (US)
(74) Representative: Forresters IP LLP

(57) **Abstract**

An auto-injector is used by a user with one hand to deliver a dose of a medication to a patient. The auto-injector includes side buttons that act as a safety lock. Depression of the side buttons applies a load to make the auto-injector ready for use to dispense the medication into the patient.

## Description

### CROSS-REFERENCE TO RELATED APPLICATIONS

This application claims priority benefit of US Application No. 18/764,773 filed July 5, 2024, which is incorporated herein by reference in its entirety.

### BACKGROUND OF THE INVENTION

With respect to anaphylaxis in children, it is reported that six million children have food allergies in the U.S. (approximately 1 in 13 children). Anaphylaxis is a severe, potentially life-threatening allergic reaction. Allergic reactions to food are responsible for 47% of anaphylaxis and 53% are unknown. The main allergens in food include peanuts (22%), tree nuts and seeds (15%) and milk and eggs (6%). The prevalence of food allergies in children is also increasing and, among children, the incidence of food allergies has doubled between 2000-2008. A likely combination of factors for the increase may include sensitization via skin in eczema patients, Vitamin D deficiency, lack of exposure to allergens in utero/first months of age, increase in allergic diseases such as asthma, allergic rhinitis, eczema, or medications during pregnancy. Evidence suggests a continued increase in childhood food allergies.

Anaphylaxis in children is a serious, potentially fatal allergic reaction with onset typically within 30-60 minutes of exposure to an allergen. The primary treatment is an epinephrine injection into a muscle. There can also be subsequent recurrences despite initial treatment hours or days later which is referred to as a biphasic reaction.

The are many disadvantages with the currently available epinephrine injection devices for patients and for pediatric patients in particular. Force is required to deliver current epinephrine doses into the child's muscle which can be intimidating for the child as the user must "wind up" their arm to get enough force to deliver the epinephrine dose into the child's muscle. With a moving or a squirming child, there is the potential for the needle to bend or to break in the child's muscle as the recommendation is for leaving the needle in the muscle for 3-10 seconds to deliver the full dose of medication. The needle size or length may be inappropriate for the child which can result in intraosseous (bone) puncture. In addition, there is a lack of continuous education about injector indication and use such that a user can go years without using a device or receiving updated refreshed education. There is a need for auto-injectors to be supported by education and teaching materials regarding indication and use.

With respect to use of current epinephrine injectors, further disadvantages include confusing instructions for use of the device with too many steps. The injectors require at least two hands to use such that one hand is required to remove the safety cap and then the device must be transferred to the other hand for injection into the patient. This can lead to confusion as to which end of the device contains the needle and can lead to accidental firing of the epinephrine dose into the user's fingers.

With current epinephrine injectors for pediatric population, there are barriers to use. Factors that contribute to not using currently available epinephrine injectors include the users having a fear of an adverse reaction in the child, fear of not using the device correctly and causing harm to the child, fear of not understanding how and when to use the device and fear of needles in general. Factors relating to the child include a "this will never happen to me" attitude, misconceptions that the child can avoid allergens, fear of bullying and a lack of confidence in the device's use and indication of use.

### SUMMARY OF THE INVENTION

In one construction, the disclosure provides an auto-injector capable of one-handed operation by a user in conjunction with a patient including a housing, a syringe assembly having a needle and a chamber for containing medication, a moveable needle cover, a safety lock preventing movement of the needle cover and at least one side button depressible when the user grips the housing with one hand to release the safety lock and to allow the needle cover to move and the needle to be inserted into the patient and medication delivered to the patient.

In another construction, the disclosure provides a method of auto-injecting a patient including the steps of depressing at least one side button by gripping the device with one hand to make the auto-injector ready for use, making contact between the ready for use auto-injector and the patient so that a needle within the auto-injector is inserted into the patient, holding the device in contact with the patient until a dose of a medication within the auto-injector is dispensed into the patient and removing the device from contact with the patient.

In another construction, the disclosure provides an auto-injector capable of one-handed operation by a user in conjunction with a patient including a housing, a syringe assembly having a needle and a chamber for containing medication, a moveable needle cover and at least one side button depressible when the user grips the housing with one hand allowing the needle cover to move on contact with the patient so that the needle can be inserted into the patient and the medication delivered to the patient.

This summary is illustrative only and should not be regarded as limiting.

### BRIEF DESCRIPTION OF THE FIGURES

The disclosure will become more fully understood from the following detailed description, taken in conjunction with the accompanying figures, wherein like reference numerals refer to like elements.
FIG. 1 is a perspective view of an auto-injector of the present invention.
FIG. 2 is a side perspective view of the auto-injector.
FIG. 3 is a bottom view of the auto-injector.
FIG. 4 is a bottom perspective view of the auto-injector including a protection sleeve.
FIG. 5 is a perspective view of the protection sleeve.
FIG. 6 is an exploded view of the interior of the auto-injector.
FIG. 7 is a perspective view of a syringe assembly and a plunger.
FIG. 8 is a sectional view taken along line 8 - 8 of FIG. 1.
FIG. 9 is a section view taken along line 9 - 9 of FIG. 8.
FIG. 10 is a view of a user removing the protective cap from the auto-injector.
FIG. 11 is a view of the user gripping the auto-injector with one hand.
FIG. 12 is a view of the auto-injector in contact with a patient.
FIG. 13A-G are views of the auto-injector in use.
FIG. 14A-E are views of an activation mechanism in use.

### DETAILED DESCRIPTION OF THE INVENTION

Before turning to the figures, which illustrate certain exemplary embodiments in detail, it should be understood that the present disclosure is not limited to the details or methodology set forth in the description or illustrated in the figures. It should be understood that the terminology used herein is for the purpose of description only and should not be regarded as limiting.

The auto-injector 20 shown and described herein according to the present invention is particularly suited for pediatric patients. For medical devices, the U.S. Food and Drug Administration defines pediatric as birth through age 21. Adult versions of auto-injectors are not always suitable for pediatric patients. The auto-injector 20 shown and described herein is equally useable by adult patients and the invention is not limited to pediatric use.

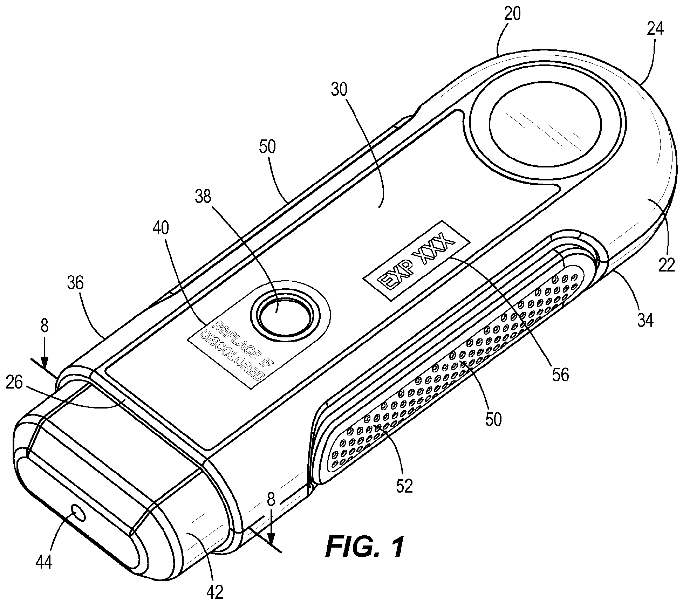

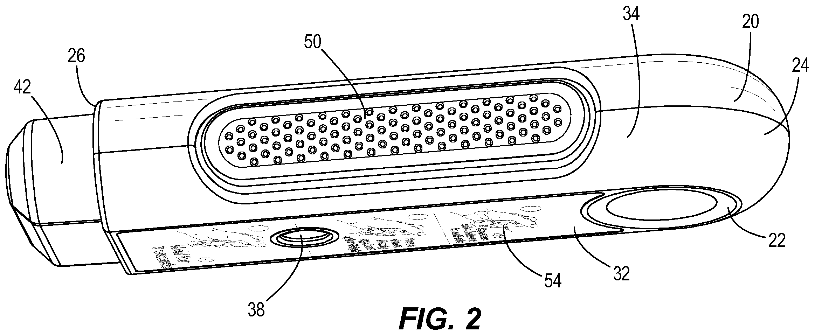

With reference to FIGS. 1-3, an auto-injector 20 is shown. The auto-injector 20 has a small, compact and convenient form factor. The auto-injector 20 has an outer housing 22 with a rounded end 24, an injection end 26 and an interior. The outer housing 22 includes two flat sides 30, 32 and two curved sides 34, 36. The length of the outer housing 22 is approximately and preferably the width of an adult's hand. The shape of the outer housing 22 enables the auto-injector 20 to be compact and easy to carry and to use. The outer housing 22 is preferably shaped to be ergonomic to hold with one hand thus allowing for a firm grip by the user and is easy to grip to prevent slippage which can occur from sweat produced by the user in an emergency situation. The outer housing 22 is preferably shaped to encourage intuitive hand-placement by the user to avoid confusion regarding which end is the injection end 26. The round end 24 provides clear direction that it is not the portion of the auto-injector that contains the needle. The outer housing 22 is preferably shaped so that most of the auto-injector 20 can be hidden by the user's hand when the user is gripping the auto-injector 20 so as to be less intimidating to patients who are scared by needles. The outer housing 22 is preferably shaped so that it does not look like a children's toy. It should be noted that other form factors and shapes of the outer housing 22 can also be utilized.

Preferably, the auto-injector 20 includes a window 38 such as one positioned on the flat sides 30 of the outer housing 22. The window 38 enables a user to view the medication contained therein to visually inspect the medication for potential discoloration and/or debris/sedimentation/particles/precipitates in the medication. Preferably, there is indicia 40 adjacent the window 38 alerting the user to the window 38 and what is shown through the window 38.

A needle cover 42 is positioned at the injection end 26 of the outer housing 22 and is partially housed in the outer housing 22. The cover 42 has therein a needed aperture 44. The cover 42 is preferably a different contrasting color from the outer housing 22 to make it clear to a user which end of the auto-injector 20 is the injection end 26.

The auto-injector 20 includes at least one side button 50. Two side buttons 50 are shown, however, any number can be utilized. One side button 50 is preferably positioned on a curved side 34, 36 of the outer housing 22 and is preferably elongate running a substantial portion of the respective curved side 34, 36. Each side button 50 can have texture 54 on its surfaces to reduce slippage of the auto-injector 20 in the user's hand. Each side button 50 can include indentations to signal user finger placement for a more intuitive grip on the auto-injector 20. Each side button 50 can be of a flared shape to make the buttons more visible and/or intuitive to press. The act of applying a force to the side buttons 50 such as by gripping firmly or by squeezing releases a safety lock on the auto-injector 20 making the auto-injector 20 ready for use. The side buttons 50 also prevent accidental medication delivery. As will be explained in more detail below, the side buttons 50 prevents a needle from moving until the side buttons 50 are pressed. The side buttons 50 can reduce the amount of load the user must apply to use the auto-injector 20. It should be noted that other types and sizes of side buttons can also be utilized such as different sized or located buttons or bars, levers and the like. Preferably, the side buttons 50 are of a different color than the curved sides 34, 36.

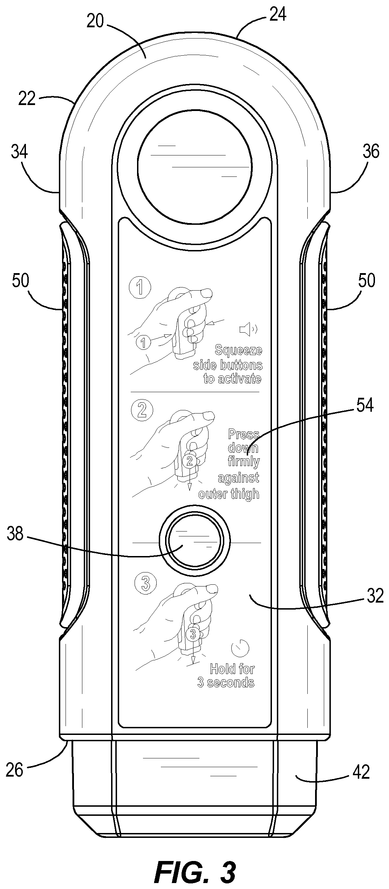

The outer housing 22 preferably has indicia 54 thereon such as text, graphics, symbols or QR codes to aid the user, especially if a user has not used or been trained in the use of the auto-injector 20. Such indicia can convey instructions for use, prompts or warnings such as that shown in FIG. 3. Each of the flat sides 30, 32 of the outer housing 22 are particularly suited to include indicia 54 for instructions and visual cues. A QR code can also be utilized to direct the user to online training resources, better onboarding materials and regular reminders of how to operate the auto-injector 20. Indicia 56 regarding the auto-injector expiration date such as printed text can be used.

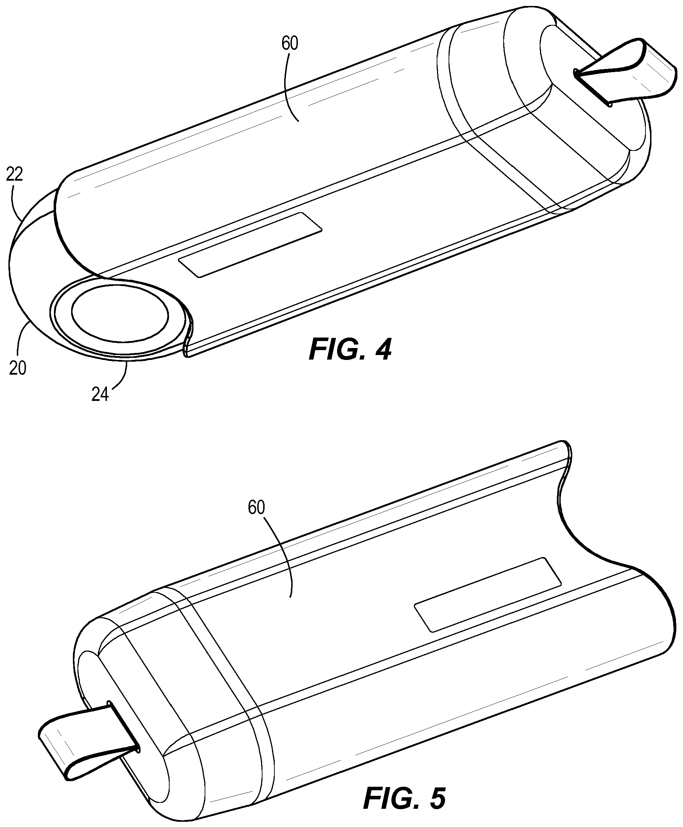

As shown in FIGS. 4-5, a protection sleeve 60 can be optionally utilized when the auto-injector 20 is being stored when not in used. The sleeve 60 reduces the chances that the side buttons 50 are inadvertently depressed, and the auto-injector 20 inadvertently actuated for use and prevents damages to the auto-injector 20. The sleeve 60 can include indica (not shown) such as personalization for a particular patient. Such personalization may include stickers with a patient's name, allergy and like personal information. It should be noted that the protection sleeve 60 is optional and plays no part in actuation of the auto-injector 20 for use. Optionally, the sleeve 60 can include an attachment loop to help easily locate the auto-injector 20 and can be clear, translucent, frosted or colored.

The auto-injector 20 preferably is designed to be easy to find to enable quick location in emergency situations. If the auto-injector 20 is located in a backpack, handbag, diaper bag or like bag, lights, sounds, music, alarms or vibrations can be utilized to quickly identify the auto-injector 20 when the bag is shaken. Such alerts will be described in more detail below. The auto-injector 20 can also have glow in the dark components for location in dark environments.

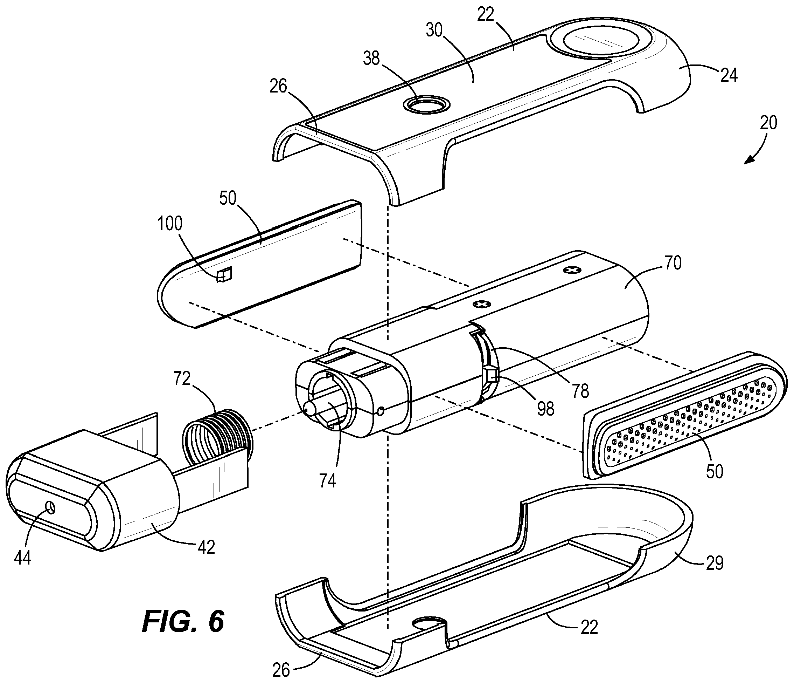

Turning now to the interior of the auto-injector, FIG. 6 and one embodiment of actuating the auto-injector 20, the auto-injector 20 includes a chassis 70 and needle guard compression spring 72. The chassis 70 engages with side buttons 50 to effectuate the safety lock as will be explained in more detail below. The chassis 70 includes a pair of slots 78.

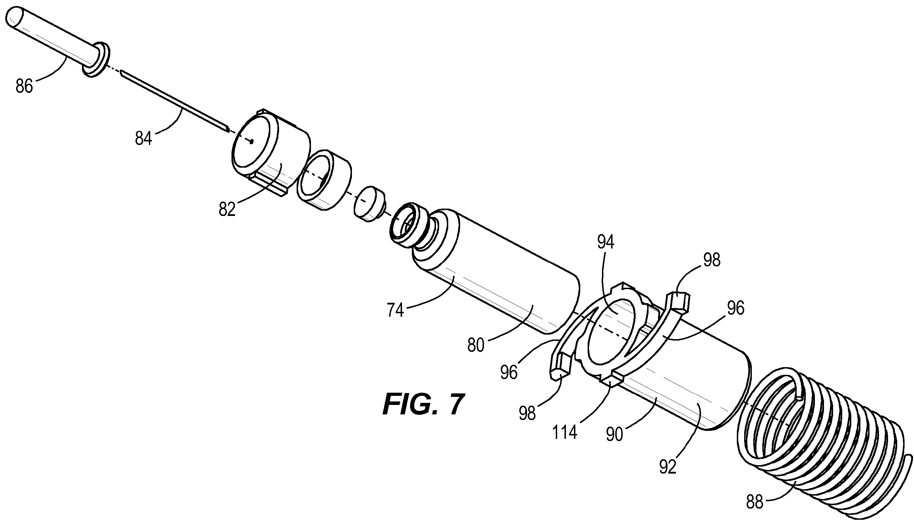

Referring to FIG. 7, a syringe assembly 74 is shown. The syringe assembly 74 is housed in the chassis 70. The syringe assembly 74 includes a chamber 80 for holding a medication. The chamber 80 can be adapted to hold any dose of medication such as a pediatric dose. Pediatric doses of medications can be in the range of, for example 0.1 mg to 0.3 mg. An infant dose is typically 0.1 mg. A dose for children between 15-65 pounds is typically 0.15 mg. The dose for children over 65 pounds is 0.3 mg. It should be noted that other doses can also be utilized. For adult patients, doses are typically 0.3 mg, however, other doses can also be injected. The medications housed in the chamber 80 can include any medications needed for patients such as epinephrine although other medications can also be dispensed. It should be noted that the auto-injector 20 is not limited to the dispensing of epinephrine.

The syringe assembly 74 includes a needle hub 82, a needle 84 and a needle boot 86. The needle 84 is appropriately sized for patient use. For example, a smaller needle can be used to accommodate a pediatric patient.

The chamber 80 is dimensioned to be housed in a plunger 90. The plunger 90 includes a cylindrical body 92, an interior bore 94 for housing the chamber 80 and a pair of curved arms 96. Each curved arm 96 terminates in an engagement member 98. Each engagement member 98 cooperates with an engagement nub 100 on the side buttons 50. The engagement nubs 100 are positioned in the slots 78 of the chassis 70. Each side button 50 engages with the engagement member 98 on the curved arm 96 as part of the safety lock as will be described below. A spring compression 88 is positioned on the outside of the cylindrical body 92.

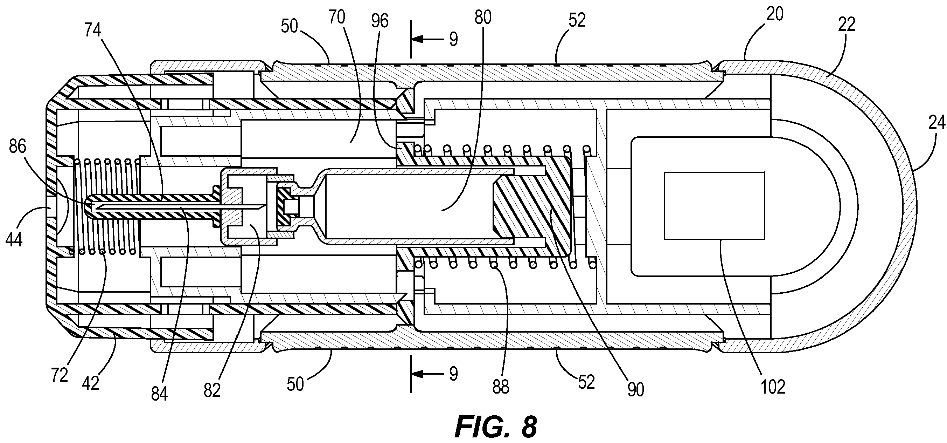

Turning to FIG. 8, optionally the auto-injector 20 can include components 102 such as a processor, memory, a speaker and a battery as are known in the art. The components 102 can be conventionally configured to provide voice and/or music for user prompts and instructions and for calming and soothing the user and patients, especially pediatric patients, as will be described below. It should be noted that the auto-injector 20 can be used without such voice and/or music as those are optionally included features.

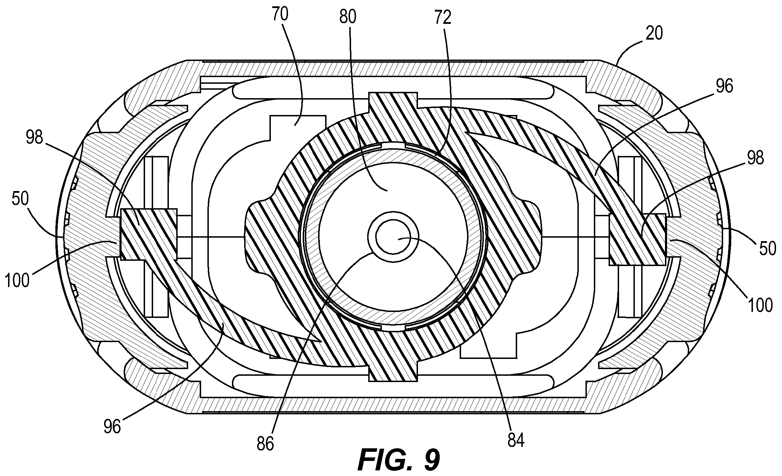

FIGS. 8 and 9 show the interior of the auto-injector 20 in assembled form.

Turning now to the use of the auto-injector 20, the user of the auto-injector 20 is typically an adult. Such an adult user may or may not have been trained in the use of the auto-injector 20. The user may also be a competent child such as one age 6 years or older.

It is important to note that use of the auto-injector 20 does not require the removal of any type of cap using a second hand by the user. The user's thumb does not push a button or other device to actuate the auto-injector 20.

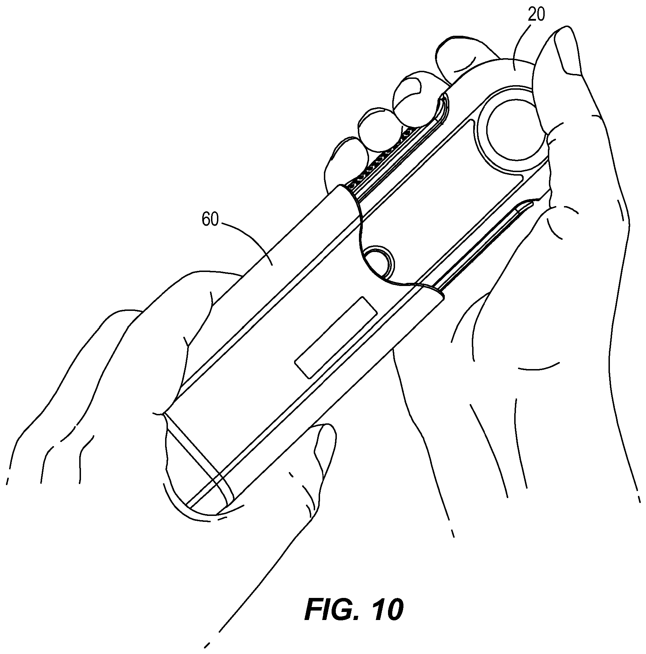

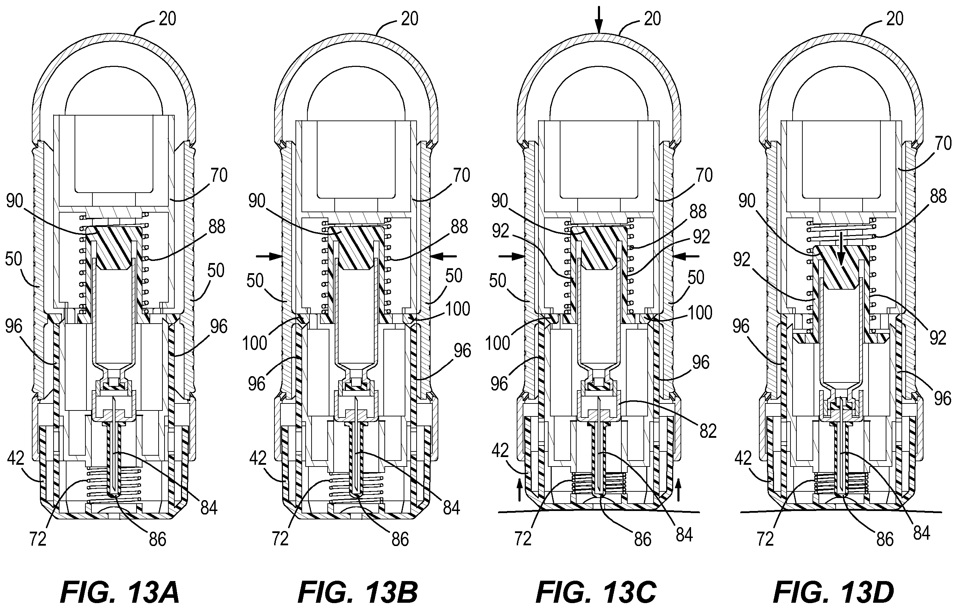

As shown in FIG. 10, if the auto-injector 20 has a protective sleeve 60, the sleeve 60 is removed. A trained or untrained user can read the instructions on the outer housing 22 or access online resources via a QR code. It should be noted that the auto-injector 20 is also intuitive for use without any training or consulting any instructions. The auto-injector 20 is now in its initial state as shown in FIG. 13A. The engagement members 98 are profiled to prevent the needle cover 42 from moving in this initial state.

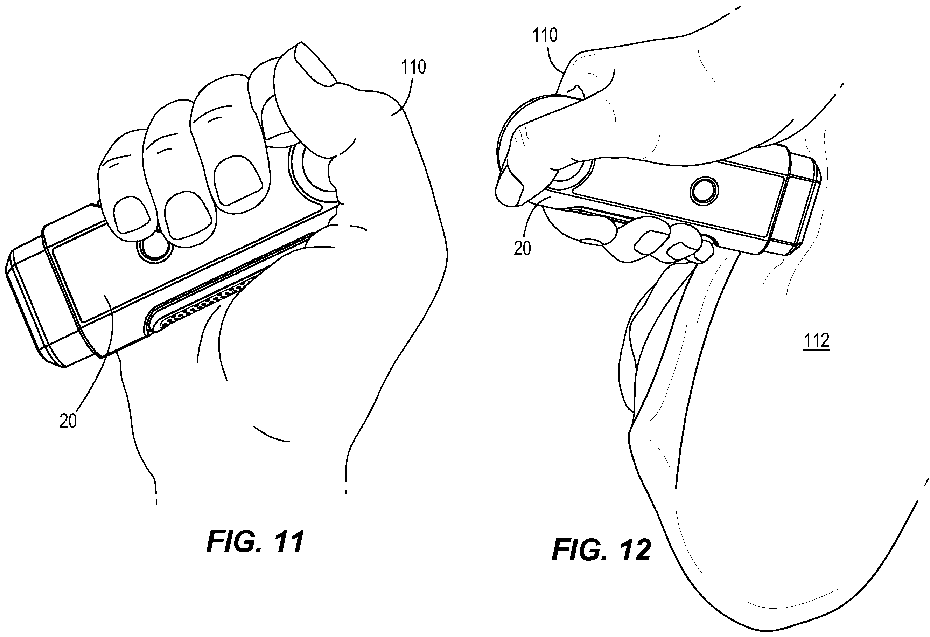

As shown in FIGS. 11 and 13B, the user grips the auto-injector 20 with one hand 110. The auto-injector 20 may have emojis, symbols, arrows, words and/or voice instructions directing the user to press or squeeze the side buttons 50. The act of firmly gripping and/or squeezing depresses the side buttons 50. The gripping motion by the user's hand 110 prevents the user from switching hands prior to use, avoiding any confusion of how to use the auto-injector 20. The gripping motion by the user's hand 110 makes it clear which end is to be brought into contact with the patient so there is no confusion as to which is the injection end 26. Optionally, when the auto-injector 20 is gripped and ready for use, a visible or audible indicator such as a light, voice, alarm or other type of signal, such as a green light or voice acknowledgement, can be utilized to communicate with the user that the auto-injector 20 is ready for use.

When the user grips the auto-injector 20 with one hand 110 and squeezes the side buttons 50, the depression of the side buttons 50 by applying a load releases the safety lock thus making the needle cover 42 moveable. Applying a load moves the arms 96 inwardly. The engagement nub 100 of each side button 50 engages the engagement member 98 on each curved arm 96 pushing each curved arm 96 inward toward the cylindrical body 92. It should be noted that other mechanisms enabling one-handed operation of the auto-injector 20 in addition to or in place of the side buttons 50 can also be utilized and that other mechanisms in addition to or in place of the side buttons 50 can enable movement of the needle cover 42.

Gripping the auto-injector 20 and thus depressing the side buttons 50 can also trigger the components 102 to broadcast slow and calming instructions of use by broadcasting voice instructions. Voice instructions delivered in this way can decrease panic in the user and the patients and can provide encouragement and confidence for the user, especially an untrained user. The voice instructions are preferably easy to follow and delivered by a soft and calming voice.

As shown in FIGS. 12 and 13C, the user brings auto-injector 20 in contact with a patient 112 by contacting the needle cover 42 with the patient 112 such as in contact with a thigh muscle. However, other locations can also be used as needed in a given situation. The auto-injector 20 can be used through the patient's clothing if need be. The auto-injector 20 may have emojis, symbols, arrows, words and/or voice instructions directing the user to inject the auto-injector20 into the patient's thigh.

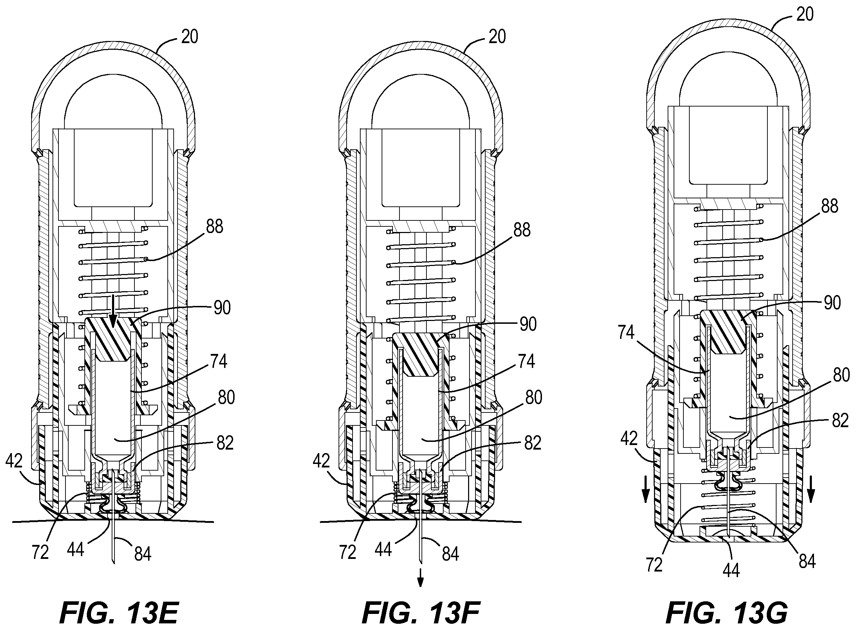

As shown in FIG. 13D, the force of the auto-injector 20 contacting the patient 112 moves the needle cover 42 inwardly against the bias of the needle guard compression spring 72. As the needle cover 42 is pushed into the interior of the outer housing 22, the engagement members 98 push the arms 96 further inside the chassis 70 until they reach a critical point and the spring 88 pushes the plunger 90 and syringe assembly 74 forwardly. As the chamber 80 starts to move, the back end of the needle 84 penetrates or pierces the chamber 80 to access the medication. As shown in FIG. 13E, as the syringe assembly 74 and the plunger 90 continue to be moved forwardly, the needle 84 moves through the needle aperture 44 and exits the needle cover 42 into the patient. As shown in FIG. 13F, once the needle hub 82 comes to rest on the chassis 70, the medication in the chamber 80 moves into the patient.

The force needed to insert the needle 84 into the patient 112 with the auto-injector 20 is reduced from other auto-injectors. The force needed with the auto-injector 20 does not require the user to perform a wind up of their arm. The reduced force can make the use of the auto-injector 20 less painful to the patient 112, especially pediatric patients. Once the needle 84 is positioned in the patient 112, voice instructions can be utilized to confirm proper placement.

The auto-injector 20 is held in the position shown in FIG. 13F over a length of a hold time, such as for example 3-5 seconds, to enable all of the medication to enter the patient. Preferably, the hold time is as short as possible, such as for example 3 seconds. It should be noted that differing hold times can also be used. Optionally, the components 102 communicates the end of the hold time to the user and the patient 112 through voice or through a song. This is reassuring to both the user and the patient 112 that the full amount of the medication has been administered and that the process of administering the medication has concluded. Optionally, a visible or audible indicator such as a light, voice, alarm or other type of signal, such as a green light or voice acknowledgement, can be utilized to communicate the end of the hold time.

As shown in FIG. 13G, when the auto-injector 20 is removed from contact with the patient 112 by the user, the spring 72 pushes the cover 42 forwardly those covering the needle 84. The auto-injector 20 can then be disposed of properly such as in a Sharps bin or like container and is decommissioned from further use. The auto-injector 20 uses a passive needle safety feature where the needle cover 42 extends past the end of the needle as the user removes the auto-injector 20 from the patient.

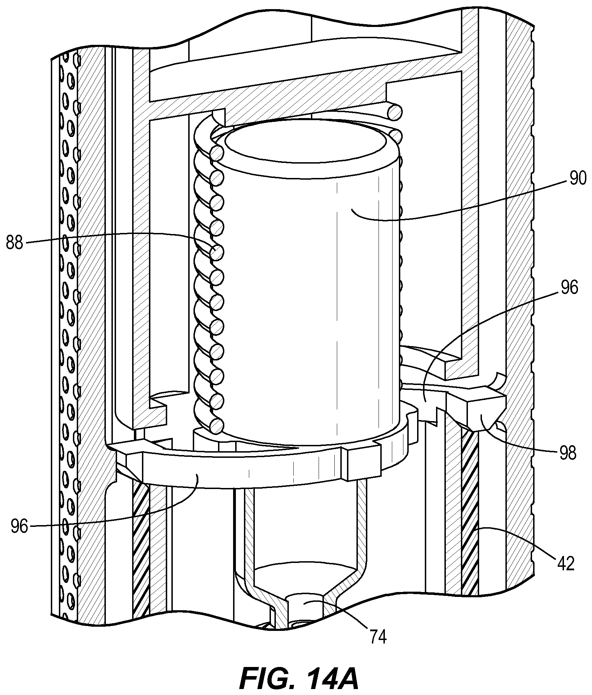

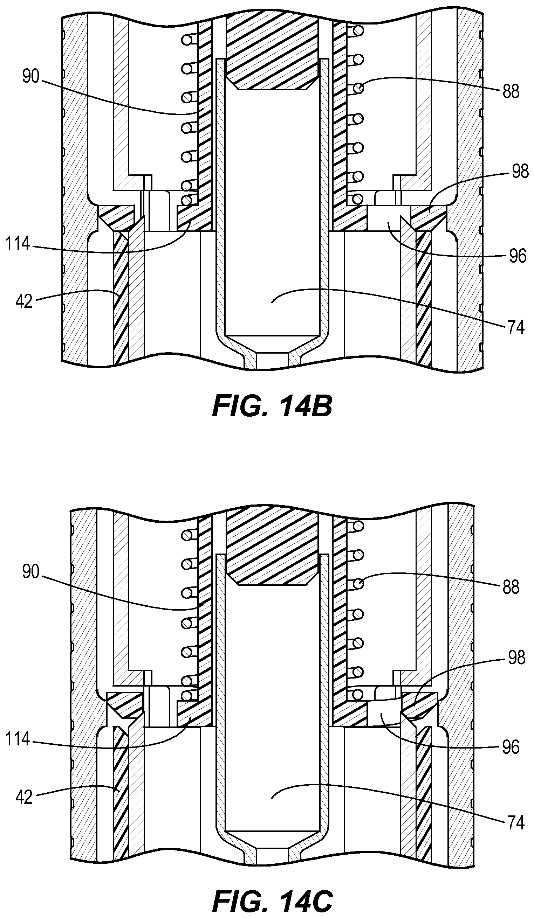

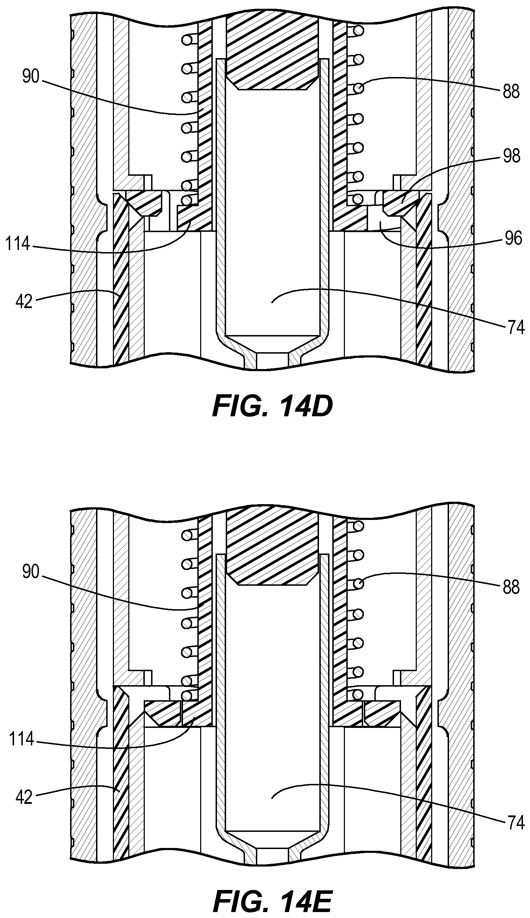

Turning specifically now to the reduced force needed to insert the needle 84 into the patient 112 with the auto-injector 20 and FIG. 14A-E, the activation is shown in detail. With reference to FIG. 14A-B, the curved arms 96 hold the plunger 90 in its stable initial state. To thereafter activate the auto-injector 20 for use, the engagement members 98 on the end of the curved arms 96 need to be pushed back, up the slope of the chassis 70. As shown in FIG. 14C, depressing the side buttons 50 moves the curved arms 96 inwardly and upwardly on the chassis 70. The needle cover 42 is prevented from moving until the curved arms 96 align with a wedge 114 on the plunger 90. As shown in FIG. 14D-E, the curved arms 96 are in a second position which thereafter allows for movement of the needle cover 42 by enabling the compression spring 88 to move the plunger 90 and the syringe assembly 74 forwardly. As shown, half of the release force of the auto-injector 20 is completed by the side buttons 50 and the remainder of the release force is completed by the needle cover 42 thus reducing the force need to use the auto-injector 20.

When used in this specification and claims, the terms "comprises" and "comprising" and variations thereof mean that the specified features, steps or integers are included. The terms are not to be interpreted to exclude the presence of other features, steps or components.

The invention may also broadly consist in the parts, elements, steps, examples and/or features referred to or indicated in the specification individually or collectively in any and all combinations of two or more said parts, elements, steps, examples and/or features. In particular, one or more features in any of the embodiments described herein may be combined with one or more features from any other embodiment(s) described herein.

Protection may be sought for any features disclosed in any one or more published documents referenced herein in combination with the present disclosure.

Although certain example embodiments of the invention have been described, the scope of the appended claims is not intended to be limited solely to these embodiments. The claims are to be construed literally, purposively, and/or to encompass equivalents.

Preferred features of the invention:
1. An auto-injector capable of one-handed operation by a user in conjunction with a patient comprising:
   a housing;
   a syringe assembly having a needle and a chamber for containing medication;
   a moveable needle cover;
   a safety lock preventing movement of the needle cover; and
   at least one side button depressible when the user grips the housing with one hand to release the safety lock and to allow the needle cover to move and the needle to be inserted into the patient and medication delivered to the patient.
2. The auto-injector of clause 1 wherein the housing has a pair of flat faces and a pair of curved faces.
3. The auto-injector of clause 2 wherein the side button is located on one of the curved faces.
4. The auto-injector of clause 1 wherein the at least one side button is two side buttons.
5. The auto-injector of clause 1 and further including a processor and speaker capable of broadcasting at least one of voice, music and alarms.
6. The auto-injector of clause 1 wherein the safety lock includes moveable curved arms.
7. The auto-injector of clause 1 and further including a passive needle safety system for use when the use of the auto-injector is terminated to cover the needle.
8. A method of auto-injecting a patient comprising the steps:
   depressing at least one side button by gripping the device with one hand to make the auto-injector ready for use;
   making contact between the ready for use auto-injector and the patient so that a needle within the auto-injector is inserted into the patient;
   holding the device in contact with the patient until a dose of a medication within the auto-injector is dispensed into the patient; and
   removing the device from contact with the patient.
9. The method of clause 8 wherein during the holding step, the auto-injector broadcasts a signal for the length of time the device should be in contact with the patient.
10. The method of clause 9 wherein the signal includes one of a voice, music and an alarm.
11. The method of clause 8 wherein the at least one side button is two side buttons.
12. The method of clause 8 wherein the side button is positioned substantially along the length of the auto-injector.
13. The method of clause 8 and further including the step of the auto-injector communicating with the patient and the user after the auto-injector is made ready for use.
14. The method of clause 13 wherein the communicating is done with one of voice and music.
15. The method of clause 8 and further including the step of making the auto-injector easy to find before activation using at least one of light, vibration, glow in the dark, voice, music and alarm.
16. An auto-injector capable of one-handed operation by a user in conjunction with a patient comprising:
   a housing;
   a syringe assembly having a needle and a chamber for containing medication;
   a moveable needle cover; and
   at least one side button depressible when the user grips the housing with one hand allowing the needle cover to move on contact with the patient so that the needle can be inserted into the patient and the medication delivered to the patient.

## Claims

1. An auto-injector capable of one-handed operation by a user in conjunction with a patient comprising:
a housing;
a syringe assembly having a needle and a chamber for containing medication;
a moveable needle cover; and
at least one side button depressible when the user grips the housing with one hand allowing the needle cover to move on contact with the patient so that the needle can be inserted into the patient and the medication delivered to the patient.

2. The auto-injector of claim 1 wherein the housing has a pair of flat faces and a pair of curved faces.

3. The auto-injector of claim 2 wherein the side button is located on one of the curved faces.

4. The auto-injector of any preceding claim wherein the at least one side button is two side buttons.

5. The auto-injector of any preceding claim and further including a processor and speaker capable of broadcasting at least one of voice, music and alarms.

6. The auto-injector of any preceding claim, further comprising a safety lock preventing movement of the needle cover, and wherein the least one side button is depressible when the user grips the housing with one hand to release the safety lock and to allow the needle cover to move and the needle to be inserted into the patient and medication delivered to the patient, and preferably wherein the safety lock includes moveable curved arms.

7. The auto-injector of any preceding claim and further including a passive needle safety system for use when the use of the auto-injector is terminated to cover the needle.

8. A method of auto-injecting a patient comprising the steps:
depressing at least one side button by gripping the device with one hand to make the auto-injector ready for use;
making contact between the ready for use auto-injector and the patient so that a needle within the auto-injector is inserted into the patient;
holding the device in contact with the patient until a dose of a medication within the auto-injector is dispensed into the patient; and
removing the device from contact with the patient.

9. The method of claim 8 wherein during the holding step, the auto-injector broadcasts a signal for the length of time the device should be in contact with the patient.

10. The method of claim 9 wherein the signal includes one of a voice, music and an alarm.

11. The method of any one of claims 8 to 10 wherein the at least one side button is two side buttons.

12. The method of any one of claims 8 to 11 wherein the side button is positioned substantially along the length of the auto-injector.

13. The method of any one of claims 8 to 12 and further including the step of the auto-injector communicating with the patient and the user after the auto-injector is made ready for use.

14. The method of claim 13 wherein the communicating is done with one of voice and music.

15. The method of any one of claims 8 to 14 and further including the step of making the auto-injector easy to find before activation using at least one of light, vibration, glow in the dark, voice, music and alarm.
